# FASCICULE DE BREVET EUROPEEN

(11) **EP 1 178 806 B1**
(45) Date de publication et mention de la délivrance du brevet: **01.09.2004**
(21) Numéro de dépôt: 00929630.2
(22) Date de dépôt: 18.05.2000
(51) Int. Cl.: A61K 31/565, A61K 9/70

(54) **DISPOSITIF TRANSDERMIQUE POUR L'ADMINISTRATION DE TESTOSTERONE OU D'UN DE SES DERIVES**
TRANSDERMALE VORRICHTUNG ZUR VERABREICHUNG VON TESTOSTERON ODER EINEM DERIVAT DAVON
TRANSDERMAL DEVICE FOR THE APPLICATION OF TESTOSTERONE OR ONE OF THE DERIVATIVES THEREOF

(30) Priorité: 19.05.1999 FR 9906367
(43) Date de publication de la demande: 13.02.2002
(73) Titulaire: PIERRE FABRE MEDICAMENT, 92100 Boulogne (FR)
(72) Inventeur: SOURNAC, Michel, F-31400 Toulouse (FR); LIORZOU, Laurent, F-31240 L'Union (FR); BOUGARET, Jo[l, F-31570 Lanta (FR)
(74) Mandataire: Warcoin, Jacques
(86) Numéro de dépôt international: PCT/FR2000/001345
(87) Numéro de publication internationale: WO 2000/071133

(56) Documents cités:
- WO-A-93/00058
- WO-A-95/01167
- WO-A-96/35427
- WO-A-98/00118
- WO-A-98/30203
- WO-A-98/37871

## Description

La présente invention concerne un dispositif transdermique auto-adhésif pour l'administration de testostérone et/ou d'au moins un de ses dérivés, un procédé de préparation de ce dispositif et l'utilisation de ce dernier pour un médicament notamment dans le cadre d'une thérapie de remplacement de testostérone.

Les dispositifs transdermiques sont des formes pharmaceutiques qui permettent l'administration percutanée de certains principes actifs, plus particulièrement dans le cadre de pathologies chroniques voire à titre préventif. Ceux destinés au traitement hormonal substitutif chez la femme ont été plus particulièrement étudiés ces dernières années, sous la forme de dispositifs transdermiques à base d'estradiol, voire associant l'estradiol à un composé à action progestative.

Dans le même ordre d'idée, sont récemment apparus, certains dispositifs favorisant l'administration d'hormones males et essentiellement ceux à base de testostérone.

WO 98 37871 décrit un dispositif transdermique pour l'administration de testostérone comprenant en particulier des promoteurs d'absorption spécifiques terpéniques.

WO 97 10812 est relatif à un système de libération transdermique de type réservoir où le principe actif est sous forme sursaturée permettant de réduire ou d'éliminer les besoins en promoteurs d'absorption.

WO 95 01167 décrit quant à lui un dispositif pour l'administration transdermique de type réservoir comprenant une combinaison d'au moins un promoteur de perméation et d'un poly-N-vinylamide, cette combinaison produisant une amélioration des flux transdermiques.

WO 96 35427 décrit également des systèmes transdermiques pour l'administration de testostérone ou un dérivé de cette dernière qui sont des systèmes transdermiques de type réservoir où le principe actif est sous forme d'une solution saturée dans un véhicule alcoolique.

Enfin le document WO 98 00118 décrit un procédé de préparation d'un dispositif d'administration transdermique de type réservoir. De la testostérone peut être mise en oeuvre dans ce dispositif où le principe actif est contenu dans une quantité au-delà de la saturation.

La testostérone connue sous la nomenclature systémique en tant que 17-hydroxyandrost-4-ène-3-one est la principale hormone circulante de type androgénique ; sous l'action de la 5 α réductase, elle se transforme en dihydro testostérone, hormone responsable de la différenciation sexuelle. Depuis de nombreuses années, la testostérone est disponible sous la forme de préparations injectables et de capsules, indiquées dans le traitement thérapeutique de l'hypogonadisme masculin. Ces formes présentent de nombreux inconvénients soit liés à des problèmes d'absorption (ou perméation au travers de la peau) soit à une mauvaise compliance du malade au traitement par voie injectable. Ainsi sont apparues les premières formes transdermiques à base de testostérone.

Parmi les dispositifs transdermiques, on distingue généralement les dispositifs réservoir des dispositifs matriciels. Dans les dispositifs réservoir, l'ingrédient actif est contenu dans un gel le plus souvent hydroalcoolique, disposé entre un film support et une membrane de contrôle. Dans le cas de dispositifs adhérant sur toute leur surface, une couche d'une matière perméable à l'ingrédient actif et adhésive - ou membrane intermédiaire de perméation - est présente entre la surface de la peau et la membrane de contrôle de la libération. Dans las autres cas, cette couche se situe en périphérie. dans les dispositifs matriciels, l'ingrédient actif est contenu dans une ou plusieurs couches matricielles (généralement à base de polymères auto-adhésifs). Dans ce cas, un contact direct avec la peau ne nécessite pas tout au moins la présence d'une membrane intermédiaire de perméation.

Un des problèmes majeurs concernant l'administration transdermique de divers principes actifs est la pénétration lente ou, en d'autres termes, la vitesse de perméation lente du principe actif à travers la peau.

Ce problème de perméation du principe actif au travers de la peau est d'autant plus crucial dans le cas d'une thérapie de remplacement de testostérone en vue d'obtenir les taux physiologiques souhaités, tout particulièrement chez l'homme où les taux physiologiques à atteindre sont naturellement supérieurs à ceux de la femme.

Ainsi, il est connu que l'administration transdermique de testostérone en quantité thérapeutique chez l'homme requiert soit la présence de plusieurs promoteurs de perméation dans le dispositif transdermique, soit la conception d'un dispositif ne pouvant être utilisé que sur la peau du scrotum, c'est-à-dire l'endroit du corps de l'homme où l'épaisseur de la peau est la plus faible, ce qui pénalise la compliance du patient au traitement.

Diverses publications font en particulier état de dispositifs transdermiques dont le réservoir ou la matrice comprend des promoteurs de perméation de la testostérone.

Par exemple, US- 5 152 997 décrit un dispositif transdermique de type réservoir, faisant appel à la combinaison de plusieurs promoteurs, type éthanol, méthyllaurate et monooléate de glycérol. Ce dispositif permet la libération d'environ 13 µg/cm²/hr de testostérone à partir d'un patch appliqué sur des zones classiques de pose de dispositif transdermique comme le bras, le ventre ou le dos.

Toutefois, comme c'est le cas pour ce dispositif de US- 5 152 997, un dispositif transdermique comprenant des promoteurs de perméation présente le plus souvent une tolérance cutanée tout à fait insatisfaisante pouvant s'expliquer par une interaction indésirable des promoteurs de perméation avec la peau.

D'autre part, il est également connu que la formulation d'un dispositif transdermique à matrice polymère auto-adhésive présente le problème de la stabilité de la testostérone dans la matrice, en particulier lorsque l'on désire avoir un état de sursaturation de la testostérone dans la matrice pour une libération de testostérone en quantité plus importante, tout particulièrement dans le cadre d'une thérapie de remplacement de testostérone chez l'homme.

Enfin, un des problèmes spécifiques des dispositifs transdermiques à matrice polymère auto-adhésive est le degré d'adhérence qui doit être suffisant pour une bonne libération du principe actif, par contact occlusif avec la peau.

On a maintenant trouvé de manière tout à fait surprenante et inattendue que le choix de certains composants permet d'obtenir un dispositif transdermique auto-adhésif présentant à la fois d'excellentes propriétés de perméation de la testostérone, de tolérance cutanée et d'adhérence, en vue d'une utilisation pour une thérapie de remplacement de testostérone notamment chez l'homme.

La présente invention a ainsi pour objet un dispositif transdermique auto-adhésif, pour l'administration de testostérone et/ou d'au moins un de ses dérivés choisis parmi ses esters ou ses dérivés ayant un substituant au moins en position 6α ou 7α, caractérisé par le fait qu'il comporte, successivement, au moins, une couche support, une couche matrice auto-adhésive et une couche protectrice détachable, ladite couche matrice comprenant, par rapport au poids total de cette dernière :
a) 40 à 80 % en poids d'au moins un polymère auto-adhésif choisi dans le groupe constitué par les polymères de type acrylique présentant un indice d'acide compris entre 10 et 70 ayant une fonctionnalité acide et une température de transition vitreuse comprise entre - 70°C et - 20°C;
b) 5 à 25 % en poids d'au moins un adjuvant de formulation pour la couche matrice choisi parmi les polymères de masse moléculaire moyenne en nombre comprise entre 2500 et 3 000 000 ;
c) 5 à 20 % en poids d'au moins un solvant de la testostérone ou de ses dérivés choisi dans le groupe constitué par le N-diéthyl-m-totuamide, le 2-octyldodécanol, le crotamiton, le dipélargonate de propylène glycol et les mélanges de ces derniers dans lesquels la solubilité de la testostérone ou de ses dérivés est comprise entre 5 et 20% en poids;
d) 2 à 10 % en poids de testostérone et/ou d'au moins un de ses dérivés, en solution sursaturée.

En particulier, l'indice d'acide du polymère auto-adhésif est de préférence compris entre 30 et 50.

De préférence, on utilisera comme polymère auto-adhésif un copolymère acrylique de masse moléculaire faible à moyenne, c'est à dire une masse moléculaire moyenne en nombre comprise entre 200000 et 400000 telle que mesurée par la technique de chromatographie par perméation de gel (GPC).

La fonctionnalité acide résulte de la présence d'acide acrylique parmi les monomères de base, lui conférant un indice d'acide tel que mesuré par les techniques classiques bien connues de l'homme du métier (notamment la technique de neutralisation).

Il faut donc comprendre que le polymère auto-adhésif utilisé pour la couche matrice du dispositif selon l'invention présente de manière caractéristique des groupes latéraux acide carboxylique libres.

De préférence, le polymère auto-adhésif est un polymère du monomère acide (méth)acrylique et d'au moins un monomère choisi dans le groupe constitué par les monomères (méth)acrylate d'alkyle en C1-C6, 2-(alkyl en C1-C6)hexyl(méth)acrylate, acétate de vinyle, (méth)acrylate de glycidyle, et 2-hydroxy(alkyl en C1-C6)(méth)acrylate.

Plus particulièrement, le polymère auto-adhésif est un polymère du monomère acide (méth)acrylique et d'au moins un monomère choisi dans le groupe constitué par les monomères (méth)acrylate de méthyle, (méth)acrylate de butyle, 2-éthylhexyl(méth)acrylate, acétate de vinyle, (méth)acrylate de glycidyle, et 2-hydroxyéthyl(méth)acrylate.

Le polymère auto-adhésif a de préférence une teneur en acétate de vinyle comprise entre 1 et 10% en poids, par rapport au poids total en monomères de base. Un tel copolymère acrylique est par exemple le DURO-TAK® 387-2052 ou 87-2052 de la société NATIONAL STARCH & Chemical qui est un adhésif autoréticulé disponible sous forme d'une solution organique de densité théorique proche de 0.92 g/cm³ et de viscosité Brookfield (à 25°C, 12 trs/min, mobile n°3) voisine de 2800 mPa.s.

Selon un mode de réalisation préféré, la couche matrice du dispositif selon l'invention est réticulée. En particulier, le polymère auto-adhésif est de préférence réticulé dans la mesure où la cohésion de la matrice résultante et donc du dispositif la comprenant s'en trouve renforcée. Un mode d'obtention d'un polymère auto-adhésif réticulé pour le dispositif selon l'invention sera en particulier décrit dans les exemples ci-après.

On citera en particulier les sels d'aluminium tel que l'acétylacétonate d'aluminium mais tout autre agent réticulant connu de l'homme du métier peut être utilisé.

Bien entendu, la couche matrice du dispositif selon l'invention peut être en particulier non réticulée, c'est à dire en particulier le polymère auto-adhésif peut ne pas être réticulé, comme l'illustre l'exemple 6 ci-après.

Parmi les adjuvants de formulation pouvant être présent dans la couche matrice du dispositif selon l'invention, choisis parmi les polymères de masse moléculaire moyenne en nombre comprise entre 2500 et 3 000 000, on peut citer les dérivés cellulosiques et plus particulièrement la carboxymethylcellulose sodique de masse moléculaire comprise entre 90 000 et 700 000, les dérivés de type alkylcelluloses comme l'hydroxyethylcellulose ou l'hydroxypropylcellulose, les polysaccharides de haut poids moléculaire et plus particulièrement les gommes xanthanes, et les polymères de type 1-vinyl-2-pyrrolidone de masse moléculaire comprise entre 2500 et 3 000 000.

En particulier, l'adjuvant de formulation est de préférence choisi dans le groupe constitué par les polymères du monomère 1-vinyl-2-pyrrolydone, les polysaccharides, les dérivés cellulosiques et les mélanges de ces derniers.

Plus précisément, l'adjuvant de formulation est de préférence choisi dans le groupe constitué par la polyvinylpyrrolidone, les gommes xanthanes, la carboxyméthylcellulose sodique et les mélange de ces derniers.

On utilisera tout particulièrement de préférence la polyvinylpyrrolidone dont la meilleure solubilité en milieu alcoolique facilite la dispersion dans une matrice adhésive du dispositif selon l'invention, compte tenu du procédé décrit ci-après pour la préparation du dispositif selon l'invention.

. Le N, N-diéthyl-m-toluamide est tout particulièrement préféré à titre de solvant de la testostérone et/ou de ses dérivés.

Selon un mode de réalisation préféré, la couche matrice du dispositif selon l'invention est telle que le polymère auto-adhésif est un polymère des monomères acide acrylique, 2-éthylhexylacrylate, acétate de vinyle et acrylate de butyle, ce polymère étant réticulé et ayant un indice d'acide compris entre 10 et 70 ainsi qu'une température de transition vitreuse comprise entre - 100°C et - 10°C, l'adjuvant de formulation est la polyvinylpyrrolidone de masse moléculaire moyenne en nombre comprise entre 44000 et 54000, et solvant est le le N, N-diéthyl-m-toluamide.

La testostérone est présente en tant que telle (17β-hydroxyandrost-4-en-3-one) dans la couche matrice du dispositif selon l'invention ou sous forme d'un de ses dérivés.

Par dérivés de la testostérone, on entend selon l'invention non seulement ses esters, tels que par exemple les formes acétate, enanthate, propionate, isobutyrate, undecanoate et cypionate, mais aussi des dérivés tels que ceux ayant un substituant au moins en position 6-α ou 7-α. En particulier, on peut citer la 7-α-méthyl testostérone, la 7-α-méthyl-19-nortestostérone, la 7-α-méthyl-11β-hydroxytestostérone, la 7-α,17-diméthyltestostérone, la 7-α,17-dimëthyl-11β-hydroxytestostérone, la 7-α,17-diméthyl-19-nortestostérone, la 7-α,17-diméthyl-11β-hydroxy-19-nortestostérone, la 6-α-méthyl testostérone, la 6-α-méthyl-19-nortestostérone, la 6-α-méthyl-11β-hydroxytestostérone, la 6-α,17-diméthyltestostérone, la 6-α,17-diméthyl-11β-hydroxytestostérone, la 6-α,17-diméthyl-19-nortestostérone, et la 6-α,17-diméthyl-11β-hydroxy-19-nortestostérone.

Selon un mode de réalisation particulièrement préféré, la couche matrice du dispositif selon l'invention comprend, par rapport à son poids total:
a) 63 à 73 % en poids d'au moins un polymère auto-adhésif des monomères acide acrylique, 2-éthylhexylacrylate, acétate de vinyle et acrylate de butyle, ce polymère étant réticulé et ayant un indice d'acide compris entre 10 et 70 ainsi qu'une température de transition vitreuse comprise entre - 100°C et - 10°C;
b) 5 à 25 % en poids de polyvinylpyrrolidone de masse moléculaire moyenne en nombre comprise entre 44000 et 54000;
c) 9 à 15 % en poids de N, N-diéthyl-m-toluamide;
d) 4 à 6 % en poids de testostérone.

La couche matrice du dispositif selon l'invention telle que décrite ci-dessus peut avantageusement comprendre en outre au moins un agent antioxydant choisi dans le groupe constitué par le butyl hydroxy Toluène (BHT), le butyl hydroxy anisole (BHA), le palmitate d'ascorbyle, l'alpha tocophérol et ses esters, l'acide citrique, le gallate de propyle et les mélanges de ces derniers.

De préférence, l'agent antioxydant est présent dans la couche matrice, par rapport au poids total de cette dernière, selon une proportion comprise entre 0,1 et 2 % en poids.

La couche support du dispositif selon l'invention doit être telle que celles généralement utilisées dans la formulation de dispositifs transdermiques, c'est à dire occlusive et inerte vis à vis des constituants de la couche matrice. Parmi les produits généralement utilisés, il faut citer les films polyéthylène, polypropylène, polyester, les complexes multicouches constitués des matières précédentes associés par exemple à de fines couches d'aluminium, les associations de copolymères d'acétate de vinyle et . d'éthylène sous forme de films ou de mousse. De préférence, on utilisera soit un film support polyester, ce dernier étant mis en oeuvre soit directement soit via un principe de transfert , soit un complexe multi couche de type polyéthylène basse densité et chlorure de polyvinylidène, ce dernier étant alors mis en oeuvre par transfert.

Avant son utilisation, le dispositif comprend bien entendu, du côté de la couche matrice, destiné à être en contact avec la peau du patient, une couche protectrice détachable qui protège la matrice adhésive. Cette couche protectrice détachable doit en particulier être constitué d'une matière présentant de bonnes propriétés de découpe, inerte vis à vis des composants de la matrice ; il faut citer parmi les produits les plus souvent utilisés, les films papier, polyester, pofychlorure de vinyle. De préférence, on utilisera un film polyester préalablement découpé afin de faciliter son retrait avant usage par le patient.

Le dispositif transdermique selon l'invention sera contenu avant utilisation dans une protection étanche de type sachet à l'aide de films complexes polyéthylène-aluminium, soit de type blister.

Le dispositif matriciel selon l'invention présente de nombreux avantages que l'on peut exposer comme suit.

Par rapport aux formes pharmaceutiques existantes, et plus particulièrement par rapport aux formes transdermiques déjà commercialisées, le dispositif selon l'invention se distingue de manière surprenante par un excellent compromis entre l'adhérence et la tolérance cutanée.

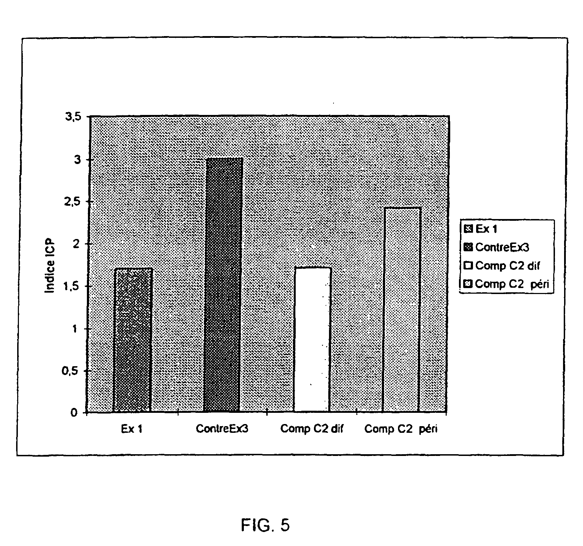

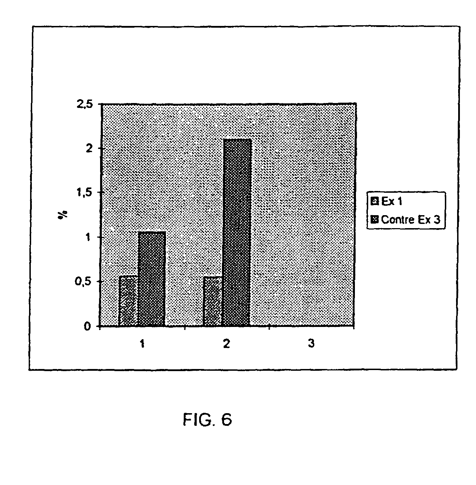

Ceci semble en grande partie résulter des caractéristiques spécifiques de la formulation matricielle adhésive à proprement parier :
a) les problèmes de solubilité de la testostérone dans l'adhésif ont été résolus grâce à la sélection de constituants plus particulièrement aptes à solubiliser de fortes quantités de principe actif. Il s'est avéré très judicieux d'utiliser le pouvoir solvant de composés organiques spécifiques tels que le DEET. En particulier, il a été possible d'incorporer dans un patch matriciel une quantité de testostérone au cm² sensiblement égale à celle présente dans des patches réservoirs, tel que le patch réservoir ANDRODERM® commercialisé par la société THERATECH (comparateur C2 ), généralement mieux adaptés à cette contrainte ;
b) comme tous les dispositifs transdermiques matriciels, le dispositif selon l'invention comprend une interface peau-patch largement saturée en principe actif, responsable du passage transcutané via un phénomène de diffusion passive. Néanmoins, compte tenu de la forte solubilité de la testostérone dans le solvant DEET, il a été nécessaire de parfaitement maîtriser le procédé de fabrication et en particulier l'étape de séchage afin de garantir un état de sursaturation avantageux. Or, si une telle sursaturation est classiquement rencontrée dans la formulation de dispositifs transdermiques, elle conduit dans. de nombreux cas à un état physico-chimique instable du principe actif, caractérisé par la survenue de phénomènes de cristallisation de ce dernier. La sélection d'adjuvants de formulation, en particulier de la polyvinylpyrrolidone, a considérablement amélioré la stabilité physico-chimique du patch sans toutefois remettre en question les caractéristiques pharmaco-techniques de ce dernier.
c) un aspect important de l'invention est que cette addition de polyvinylpyrrolidone a conduit à améliorer la tolérance du produit fini, ce qui constitue un avantage en terme de compliance du malade au traitement et d'efficacité ( voir figure 5 );
d) un autre aspect important de l'invention est que cette addition de polyvinylpyrrolidone a conduit à améliorer la stabilité-physico chimique du principe actif dans la couche matrice adhésive. Ainsi, cette couche matrice, dont le caractère acide n'est pas à priori favorable à la stabilité de la testostérone, est capable de manière surprenante de préserver la dégradation de la testostérone, dans des conditions de stabilité (voir figure 6 );
e) un aspect particulièrement avantageux de l'invention est la présence simultanée de DEET et de PVP;
f) enfin, il faut noter que les performances spectaculaires du dispositif matriciel selon l'invention sont obtenues sans devoir recourir à des agents promoteurs de perméation en tant que tels de la testostérone qui, comme déjà indiqué ci-dessus, sont généralement plus ou moins bien tolérés et qui, compte tenu des conditions particulières d'une administration occlusive, peuvent être responsables de phénomènes d'intolérance cutanée. Ainsi, le dispositif selon l'invention peut avantageusement être exempt d'agent(s) promoteurs de perméation en tant que tel(s). En outre, la non nécessité d'agents promoteurs de perméation est également un avantage considérable dans la mesure où ces composés sont dans la plupart des situations susceptibles de plastifier les adhésifs dans lesquels ils sont incorporés et ainsi de modifier considérablement les propriétés intrinsèques de ces derniers. Dans ces cas, l'homme du métier doit recourir à l'emploi d'agents correctifs de type tackyfiant qui ne sont donc pas non plus nécessaires à la réalisation du dispositif selon l'invention d'où une économie avantageuse de temps et d'argent.

Les dispositifs transdermiques selon l'invention sont réalisés suivant les techniques généralement employées par l'homme de l'art; ces techniques sont celles du mélange, de l'enduction, du séchage, du contrecollage, de la refente et de la découpe.

La présente invention a également pour objet un procédé de préparation du dispositif transdermique tel que décrit ci-dessus.

Le procédé selon l'invention est caractérisé en ce qu'il comprend les étapes consistant :
a) à réaliser, dans un mélangeur, sous agitation, une solution de testostérone ou d'un de ses dérivés dans un premier solvant de fabrication, le tetrahydrofurane (THF);
b) à incorporer, dans la solution ainsi obtenue, un deuxième solvant de fabrication, choisi parmi l'éthanol, l'isopropanol ou un mélange de ces derniers, ainsi que le solvant de la testostérone ou de son dérivé tel que défini plus haut, destiné à être présent dans le dispositif, de préférence la N-N-diéthyl-m-toluamide (DEET);
c) à incorporer progressivement, sous agitation, dans un mélangeur distinct, homogénéiseur, l'adjuvant de formulation pour la couche matrice, de préférence la polyvinylpyrrolidone, au polymère auto-adhésif; puis
d) à incorporer le mélange obtenu à la fin de l'étape b) dans celui obtenu à l'étape c) de manière à obtenir un mélange final homogène; et
e) à enduire le mélange homogène ainsi obtenu, à une température comprise entre 50°C et 110°C sur un support final de type polyester ou un support intermédiaire provisoire antiadhérent, à raison de 50 à 150 g/m², grammage mesuré sur le film sec;
f) à sécher l'enduction ainsi obtenue, par séchage progressif à une température comprise entre 50°C et 110°C, afin d'évaporer les solvants de fabrication (par exemple THF et alcool) et, le cas échéant, permettre la réticulation du polymère adhésif.

Des variantes au procédé tel que précédemment décrit sont préconisées et caractérisées par :
- la réalisation d'un prémélange distinct contenant la PVP et l'éthanol ;
- la suppression du THF et son remplacement par de l'éthanol;
- l'emploi d'un séchage de type infra rouge court.

Avantageusement, il a été mis en évidence la forte solubilité de la testostérone dans le tetrahydrofuranne ainsi que la compatibilité de ce solvant très polaire avec les principaux composants de la formulation.

Compte tenu de cette forte solubilité de la testostérone dans le THF, qui est de l'ordre de 27% m/m, la mise en solution sous agitation de l'étape a) ne présente pas de difficulté particulière. Par ailleurs, dans le cadre de la présente invention, on utilisera de préférence une solution de testostérone dans le THF afin de faciliter le mélange du principe actif dans le copolymère acrylique adhésif.

L'étape c) doit conduire à la réalisation d'un mélange homogène débarrassé de tout grumeau, ce qui constitue l'une des caractéristiques de ce procédé. Ce dernier peu être conduit à température ambiante, ce qui présente un avantage par rapport à d'autres techniques par exemple dites "hot melt" qui sont généralement plus stressantes vis à vis du principe actif.

En fin d'enduction, le film séché est contrecollé sur un film support. Selon le cas, on transfère alors le film obtenu à l'étape précédente sur le support final sélectionné. L'enduction obtenue doit généralement subir des étapes de refente puis de découpe finale des dispositifs avant conditionnement selon l'un ou l'autre des systèmes possibles, sachet ou blister.

Enfin, la présente invention a également pour le dispositif transdermique, tel que décrit ci-dessus, pour son utilisation en tant que produit thérapeutiquement actif dans un médicament.

L'invention a en particulier pour objet l'utilisation du dispositif transdermique, tel que décrit ci-dessus, pour la préparation d'un médicament destiné à une thérapie de remplacement de testostérone.

Un tel médicament est destiné à une thérapie journalière, hebdomadaire ou bi hebdomadaire de remplacement de testostérone.

Plus particulièrement, l'invention a pour objet l'utilisation du dispositif transdermique, tel que décrit ci-dessus, comme médicament destiné au traitement et/ou à la prévention de l'hypogonadisme, au traitement et/ou à la prévention des troubles de l'andropause, au traitement et/ou à la prévention de l'impuissance sexuelle, au traitement et/ou à la prévention des troubles de la fertilité chez l'homme, au traitement et/ou à la prévention de l'oligospermie et, enfin, au traitement et/ou à la prévention de l'éjaculation précoce.

Dans ce qui suit, les exemples sont destinés à illustrer la présente invention et ne doivent en particulier en aucun cas être interprétés comme pouvant en limiter la portée.

D'autre part, les figures présentées en annexe sont explicitées ci après.

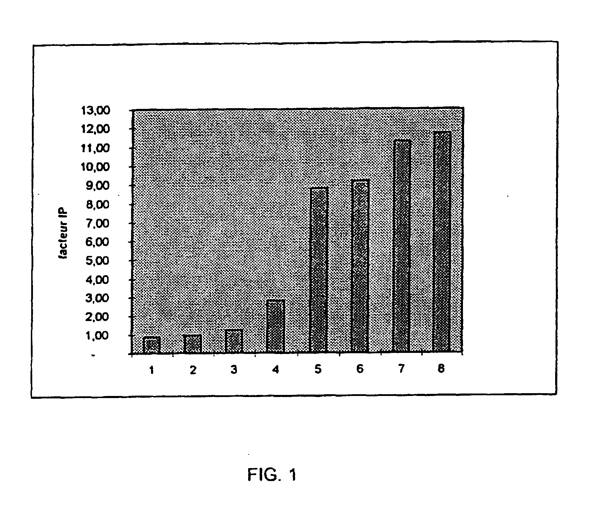

La figure 1 représente les indices de perméation (IP) aussi appelés « enhancement factor « qui permettent de comparer in vitro l'effet promoteur de différents produits.

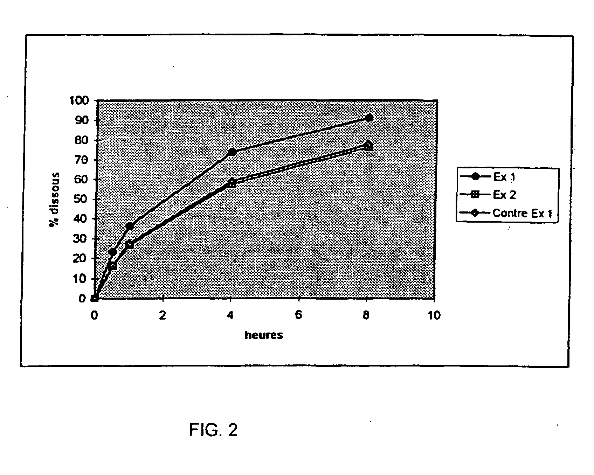

La figure 2 représente les quantités libérées in vitro de testostérone en fonction du temps dans le cas des différentes formulations des exemples 1 et 2 selon l'invention et du contre exemple 1.

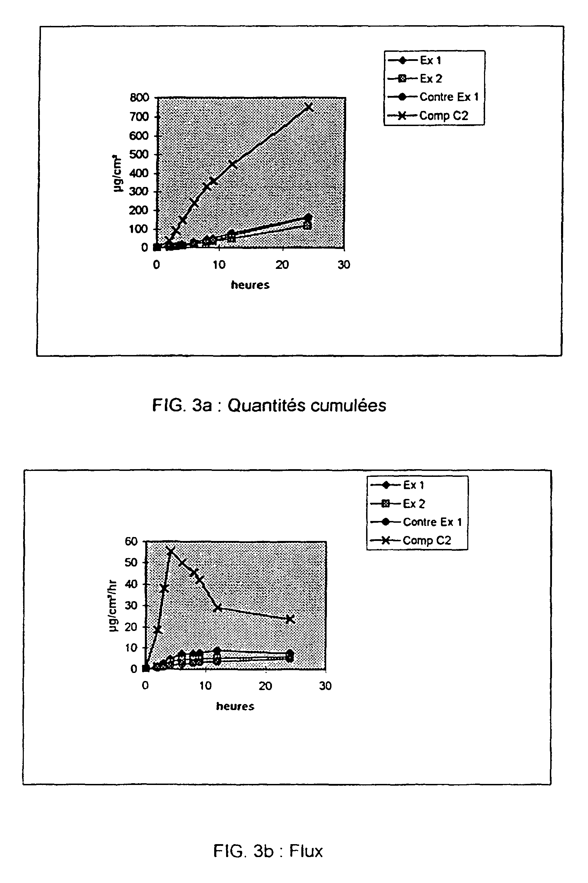

Les figures 3a et 3b représentent les quantités libérées de testostérone ex vivo sur peaux animales soit en quantité cumulée (µg/cm²) soit en flux (µg/cm²/hr) dans le cas des différentes formulations des exemples 1 et 2 selon l'invention et du contre exemple 1, et dans le cas d'un dispositif transdermique antérieurement connu et commercialisé sous la marque ANDRODERM® par la société THERATECH (comparateur C3).

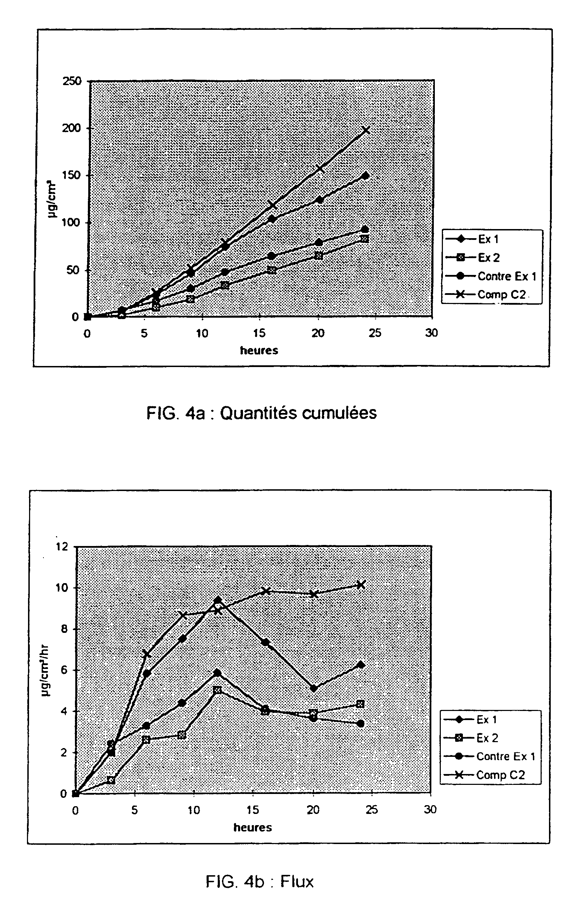

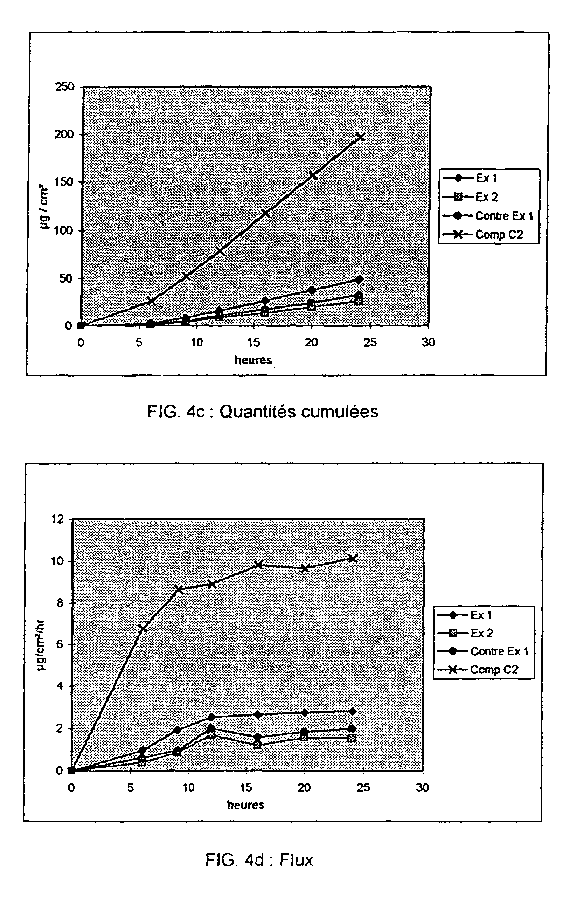

Les figures 4a à 4d représentent les quantités libérées de testostérone ex vivo sur peaux humaines soit en quantité cumulée (µg/cm²) soit en flux (µg/cm²/hr) dans le cas des formulations des exemples 1 et 2 selon l'invention et du contre exemple 1, et dans le cas d'un dispositif transdermique antérieurement connu et commercialisé sous la marque ANDRODERM® par la société THERATECH (comparateur C2).
La figure 5 représente les indices d'irritation cutanée primaire qui permettent de comparer en première intention la tolérance de différentes formulations.
La figure 6 représente les quantités de produits de dégradation décelées dans deux compositions qui différent par la présence ou non de l'adjuvant de formulation polyvinylpyrrolidone.

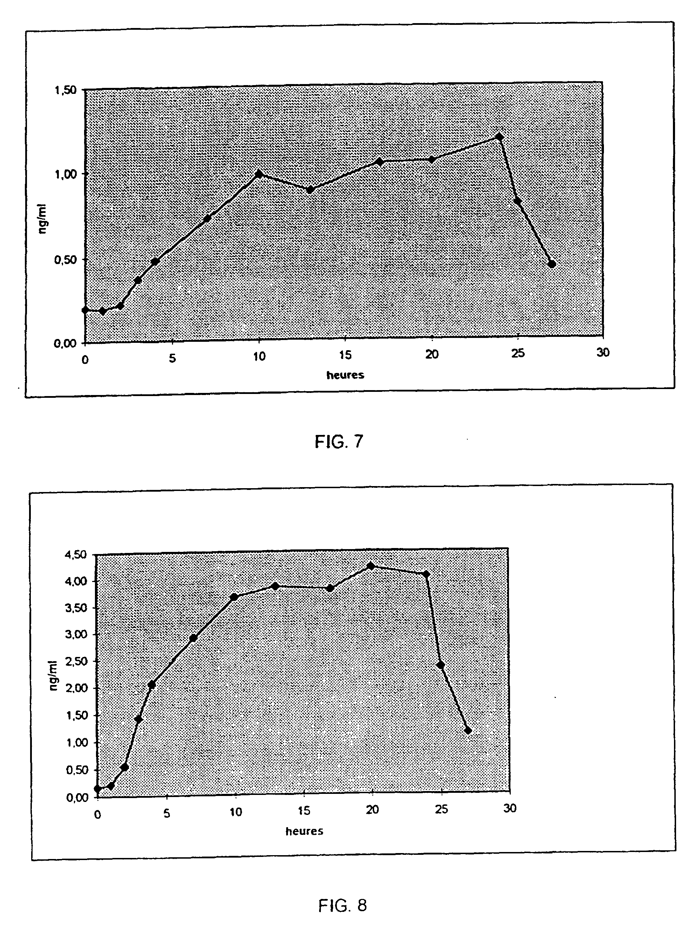

La figure 7 représente les taux plasmatiques moyens de testostérone obtenus chez 5 femmes ménopausées après administration pendant 24 heures d'un patch selon l'invention.
La figure 8 représente les taux plasmatiques moyens de testostérone obtenus chez 5 femmes ménopausées après administration pendant 24 heures de deux patches selon l'invention.

### Exemple 1 : préparation d'un patch selon l'invention.

On introduit dans un mélangeur 72 g de testostérone et 271 g de tétrahydrofurane, et on agite à température ambiante pendant au moins 15 minutes . Puis une partie aliquote de la solution obtenue est isolée, soit un prélèvement de 70 g auxquels sont rajoutés 45 g d'ethanol et 38 g de DEET. Ce prémélange est conservé jusqu'à sa prochaine utilisation.

Dans un mélangeur homogénéiseur également container de transfert, on introduit 413 g de copolymère acrylique commercialisé par la société NATIONAL STARCH & Company sous le nom DURO-TAK 387-2052®. Puis sous agitation permanente et sous vide, on rajoute progressivement par petites quantités de la polyvinylpyrrolidone, commercialisée sous le nom de KOLLIDON K30 par la société BASF. La polyvinylpyrrolidone n'est pas facilement soluble dans le milieu solvant du copolymère acrylique, et à ce stade de la fabrication, on constate que l'addition du prémélange initial améliore significativement la capacité de la polyvinylpyrrolidone à se disperser de manière homogène dans l'adhésif.

On maintient l'agitation jusqu'à obtention d'un mélange homogène, puis si nécessaire on laisse dégazer le mélange pendant environ 30 minutes.

On transfère ce mélange final vers la trémie d'alimentation d'une tête d'enduction qui permet de déposer sur un support polyester siliconé une quantité de l'ordre de 120 ± 10 g/m². On sèche en appliquant un gradient de température compris entre 50°C et 100°C afin d'évaporer les solvants de fabrication et permettre la réticulation de l'adhésif acrylique. En règle générale, il est souhaitable de parfaitement maîtriser cette étape qui contribue largement à la qualité du produit fini ; selon la dite invention, à ce gradient de température est associé la possibilité de faire varier le mode de séchage, en appliquant préférentiellement dans un premier temps un mode dit « air chaud à jet important « puis dans un second temps un mode dit « infra rouge court « . En fin de séchage, la matrice adhésive à l'état sec est contrecollée sur un film support polyester. Les rouleaux ainsi obtenus sont ensuite refendus en produits intermédiaires de plus faible largeur, eux mêmes ensuite découpés selon la dimension désirée du produit fini. Ces derniers sont ensuite conditionnés soit dans des sachets, soit dans des blisters.

### Exemple 2 : préparation d'un patch selon l'invention.

On procède de façon analogue à l'exemple 1 , en remplaçant le copolymère acrylique DURO-TAK 387-2052 par un copolymère acrylique auto-réticulable, sous la forme d'une solution à environ 28 % p/v de Butylacrylate, de 2-Ethylhexylacrylate, d' Acide Acrylique , de 2-hydroxyethylacrytate , de methylmethacrylate et, en tant qu'agent de réticulation, d'acétylacétonate d'aluminium et de t-amylperoxypivolate, ledit copolymère adhésif « prêt à l'emploi » ayant une température de transition vitreuse de - 26°C et étant commercialisé par la société NATIONAL STARCH & Chemical sous le nom DURO-TAK 87-2074®.

### Exemple 3 : préparation d'un patch selon l'invention

On procéde de façon analogue à l'exemple 1, en réduisant la quantité du solvant du principe actif, soit une quantité de N-N-diéthyl-m-toluamide dans le mélange avant enduction, de l'ordre de 6%.

### Exemple 4 : préparation d'un patch selon l'invention

On procède de façon analogue à l'exemple 1, en remplaçant la polyvinylpyrrolidone par un autre agent polymérique, de préférence la carboxymethylcellulose sodique, commercialisée sous le nom de CARMELLOSE® sodique par la société AQUALON.

### Exemple 5 : préparation d'un patch selon l'invention

On procéde de façon analogue à l'exemple 1, en remplacant la polyvinylpyrrolidone par un autre agent polymérique, de préférence une gomme xanthane, commercialisée sous le nom de KELTROL CR® par la société KELCO.

### Exemple 6 : préparation d'un patch selon l'invention

On procède de façon analogue à l'exemple 1 , en remplaçant le copolymère acrylique DURO-TAK 387-2052 par un copolymère acrylique non auto-réticulable, sous la forme d'une solution à environ 28 % p/v de Butylacrylate, de 2-Ethylhexylacrylate, d'Acide Acrylique, de 2-hydroxyethylacrylate , de methylmethacrylate ledit copolymère adhésif « prêt à l'emploi » ayant une température de transition vitreuse de - 26°C et étant commercialisé par la société NATIONAL STARCH & Chemical sous le nom DURO-TAK 87-2051 ®.

### Contre exemple 1

On procède de façon analogue à l'exemple 2 , en ajoutant dans le prémélange actif un promoteur de perméation, sous la forme de 3% de laurate de sorbitan ( CAS n° 1338-39-2) commercialisé par la société SEPPIC sous le nom MONTANE 20 ®.

### Contre Exemple 2

On procède de façon analogue à l'exemple 2 , en ajoutant dans le prémélange actif un promoteur de perméation , sous la forme de 3% phospholipides de soja commercialisés par la société LUCAS MEYER sous le nom de PRO-LIPO 242B®, ou par la société NATTERMANN sous le nom de PHOSAL 50PG®.

### Contre Exemple 3

On procède de façon analogue à l'exemple 1 mais la composition décrite ne comporte pas de dérivé polymérique supplémentaire autre que l'adhésif.

### Comparateur C1

Il s'agit du produit commercialisé par la société ALZA sous le nom de marque TESTODERM CIII®. ce dispositif transdermique matriciel à application scrotale est dépourvu d'agents promoteurs et, par conséquent, est plus particulièrement adapté à la conduite de protocoles in vitro destinés à sélectionner les meilleurs promoteurs de perméation de ce principe actif.

### Comparateur C2

Il s'agit du dispositif commercialisé sous la marque ANDRODERM 5MG/DAY®par la société THERATECH ou de la version délivrant uniquement 2.5 mg / jour . Ce dispositif réservoir est constitué par un gel hydroalcoolique de testostérone à base d'hydroxypropylcellulose , renfermant des agents promoteurs de perméation tels que le méthyllaurate et le glycérolmonooléate . La surface diffusante constituée d'une membrane de contrôle est centrée autour d'une partie périphérique adhésive qui permet le maintien du patch sur une zone d' application non scrotale.

### Comparateur C3

Il s'agit du comparateur C2 mais limité à la partie gel hydroalcoolique contenant le principe actif, c'est à dire débarrassé de la membrane de contrôle du flux et de la couronne adhésive.

Au cours du développement du dispositif transdermique selon l'invention, plusieurs essais ont été nécessaires.

Des études de sélection des composants de la matrice adhésive hors adhésif à proprement parler sont utiles et mettent en oeuvre des tests de solubilité et de perméation in vitro. Pour évaluer le pouvoir solubilisant de différents composants, des études de solubilité à saturation sont réalisées.

L'analyse du tableau I ci-après permet d'identifier plusieurs catégories de produits, à savoir :
- des produits capables de solubiliser des fortes quantités de testostérone (> 20 % m/m)qui sont aussi des cosolvants usuels de polymères acryliques adhésifs, comme l'éthanol et le THF;
- d'autres produits capables de solubiliser des quantités comprises entre 4 et 15 % m/m de testostérone et qui sont des promoteurs de perméation cités pour certains principes actifs dont les esters de sorbitan ou le N-N-diéthyl-m-toluamide;
- des produits moins intéressants pour lesquels la solubilité du principe actif n'excède pas 4% m/m.

**Tableau I**

| solubilité à saturation de la testostérone dans | |
|---|---|
| : | %m/m |
| Cyclohexane | 0,06 |
| Methyllaurate | 1,43 |
| Polysorbate 80 | 2,62 |
| Sorbitan laurate | 4,12 |
| Acétate d' ethyle | 4,45 |
| Lysophosphatidylcholine | 6,96 |
| Isopropanol | 12,80 |
| N-N-diéthyl-m-toluamide | 13,95 |
| Lauryl pyrrolidone | 15,90 |
| Ethanol | 22,12 |
| Tetrahydrofuranne | 27,01 |

En ce qui concerne la seconde catégorie de produits, il est plus particulièrement intéressant, de vérifier leur effet promoteur de perméation vis à vis du principe actif selon l'invention. Des études de perméation ex vivo sur peaux animales sont mises en oeuvre à l'aide d'un produit de référence dont la cinétique constitue un "blanc" puis après prétraitement préalable de la peau avec le composé promoteur. Le rapport de la quantité cumulée à 24 hrs de l'essai par rapport au "blanc" conduit au facteur indice de perméation "IP" ou "enhancement factor". Un indice de perméation proche de 1 signifie que le produit testé n'a pas d'effet promoteur pour le principe actif en question et dans les conditions opératoires mises en oeuvre. Un classement est alors possible selon les indications du Tableau Il ci après.

**Tableau II**

| Indice de perméation | |
|---|---|
| 2 octyl dodécanol | 0,86 |
| DEET | 0,95 |
| Methyl laurate | 1,24 |
| Myristate de propylène glycol | 2,81 |
| Mono oléate de glycérol | 8,79 |
| Lyso phosphatidylcholine | 9,18 |
| Laurylpyrrolidone | 11,27 |
| Sorbitan Laurate | 11,70 |

L' analyse des informations contenues dans les Tableaux I et II permet la sélection des composants de la couche matrice adhésive et plus précisément celle des composés à la fois solvants de la testostérone et co solvants du polymère acrylique adhésif en association avec d'autres solvants comme le N-N -diéthyl-m-toluamide dépourvu d'effet promoteur de la testostérone. Cette étape a ainsi permis d'incorporer dans un dispositif transdermique de type matriciel une quantité de principe actif proche de celle contenue dans le comparateur C2 patch réservoir ANDRODERM®, et supérieure à 0.5 mg/cm².

Les rendements des dispositifs selon l'invention sont généralement déterminés à partir des mesures des quantités d'hormone libérées en 24 heures sur un modèle de peau ex-vivo, soit d'origine animale, soit d'origine humaine. Dans le premier cas, il s'agit de quantifier la quantité de testostérone libérée par un dispositif transdermique d'une surface de 4,4 cm² , déposé à la surface d'un échantillon de peau abdominale de souris « nude « mâle, dans une cellule statique type cellule « de Franz » dont le compartiment récepteur contient 22 ml d'une phase réceptrice eau (NaCl 0.9%) - éthanol (75/25) maintenue à 32°C. On effectue des prélèvements dans le compartiment récepteur à 2, 3, 4, 6, 8, 9, 12, et 24 heures puis on dose un aliquot par chromatographie liquide. Les essais sont réalisés pour un même essai sur 5 échantillons de peau et les résultats indiqués dans le Tableau III reflétent les quantitées moyennes libérées exprimées sous la forme d'un flux ( µg) par unité de surface (cm²) et unité de temps (hr) :

**Tableau III**

| | Comparaison de flux ( µg / cm² / hr ) | | | | | | | | |
|---|---|---|---|---|---|---|---|---|---|
| | 0 | 2 h | 3h | 4h | 6h | 8 h | 9 h | 12 h | 24 h |
| Ex 1 | 0 | 1,4 | 2,73 | 4,90 | 7,07 | 7,2 | 7,41 | 8,73 | 7,49 |
| Ex 2 | 0 | 1,0 | 2,02 | 3.40 | 4,69 | 4,62 | 4,62 | 5,2 | 6,01 |
| Contre Ex 1 | 0 | 0,5 | 0,97 | 1,61 | 2,35 | 2,75 | 3,19 | 3,65 | 5,03 |
| Camp C2 | 0 | 18,6 | 38 | 55,45 | 50,0 | 45,48 | 42,10 | 28,96 | 23,64 |

Ces essais permettent à la fois de différencier les caractéristiques de libération de différentes formulations de type matricielle et de visualiser les différences de cinétiques vis à vis d'un autre dispositif de type réservoir. En règle générale, il est préférable de conduire le même genre d'essais en utilisant de la peau humaine provenant de biopsies chirurgicales. En effet, les différences de perméabilité cutanée inter espèces sont connues comme ne permettant pas de prédire avec suffisamment de fiabilité les quantités qui seront libérées in vivo à partir d'un seul test de perméation réalisé sur peau animale. Dans ce cas, il s'agit de quantifier la quantité de testostérone libérée par un dispositif transdermique d'une surface de 5 cm² , déposé sur un échantillon de peau humaine, de surface 2,02 cm² provenant de chirurgie esthétique, conservée à - 20°C, décongelée à + 4°C puis dermatomée à 350 µm, dans une cellule statique type cellule « de Franz » dont le compartiment récepteur contient 4 ml d'une phase réceptrice eau ultra pure - éthanol (75/25) maintenue à 37°C. On effectue des prélèvements dans le compartiment récepteur à, 3, 6, 9, 12 , 16, 20 et 24 heures puis on dose un aliquot par chromatographie liquide .Les essais sont réalisés pour un même essai sur 5 échantillons de peau et 2 donneurs différents ; les résultats indiqués dans le Tableau IV reflètent les valeurs moyennes obtenues à propos des quantités libérées exprimées sous la forme des quantités cumulées pour le donneur n°1 :

**Tableau IV**

| | Comparatif Quantités Cumulées µg / cm² | | | | | | | |
|---|---|---|---|---|---|---|---|---|
| | 0 | 3 h | 6h | 9h | 12 h | 16 h | 20 h | 24 h |
| Ex 1 | 0 | 5,99 | 23,49 | 46,05 | 74,2 | 103,47 | 123,78 | 148,68 |
| Ex2 | 0 | 1,96 | 9,75 | 18,22 | 33,24 | 49,07 | 64,56 | 81,83 |
| Ex 3 | 0 | 7,19 | 16,96 | 30,09 | 47,64 | 64 | 78,46 | 91,85 |
| Comp C2 | 0 | 6,0 | 26,32 | 52,23 | 78,88 | 118,14 | 156,77 | 197,29 |

Les résultats du Tableau IV montrent que l'une des formulations (exemple 1) selon l'invention permet la libération de quantités de testostérone à 24 heures supérieures à celles libérées par le comparateur C2. Ce dernier dispositif est caractérisé par une surface de libération active de 15 cm² dont le produit par la quantité cumulée par cm² à 24 heures permet le calcul de la quantité qui serait théoriquement libérée in vivo, soit 2,95 mg / 24 heures. En ce qui concerne le dispositif matriciel selon l'invention, et plus particulièrement le dispositif décrit à l'exemple 1, le même produit de la quantité cumulée par cm² à 24 heures par une surface théorique de 40 cm² permet le calcul de la quantité qui serait théoriquement libérée in vivo, soit 5,94 mg / 24 heures. En tenant compte des surfaces réelles des dispositifs (40 cm² pour les formulations des exemples 1 à 3, et 15 cm² pour le Comparateur C2), on constate l'obtention de quantités libérées supérieures pour les formulations de la présente invention.

On a enfin réalisé sur les dispositifs matriciels selon l'invention des tests destinés à caractériser la libération in vitro de testostérone à partir de ces dispositifs. Ces tests généralement adaptés à partir des recommandations de diverses Pharmacopées, dont la Pharmacopée américaine sont appelés tests de dissolution. Chaque réacteur composant l'appareil de dissolution "à palettes tournantes" est équipé d'un verre de montre sur lequel est placé le dispositif à tester à l'aide d'un ruban adhésif double face. Un milieu de dissolution composé du mélange eau / éthanol (90/10) est introduit dans chaque réacteur, chauffé à 37°C, agité à 50 trs /min. Des prélèvements sont réalisés plus particulièrement aux temps 30 min, 1, 4, et 8 heures à partir desquels les dosages sont réalisés par chromatographie liquide. Le tableau V ci après présente les valeurs moyennes obtenues à partir de 6 essais réalisés sur le même dispositif.

**Tableau V**

| Comparatif dissolution in vitro % | | | | | |
|---|---|---|---|---|---|
| | 0 | 0,5 h | 1 h | 4h | 8h |
| Ex 1 | 0 | 23,30 | 36,30 | 73,90 | 91,10 |
| Ex 2 | 0 | 16,30 | 26,90 | 57,50 | 76,30 |
| Contre Ex 1 | 0 | 16,50 | 27,60 | 58,90 | 77,80 |

Ce test permet essentiellement de caractériser la libération in vitro dans un souci de contrôle de routine du dispositif matriciel. Il est plus particulièrement pertinent de mettre en évidence des tendances identiques entre les profils de dissolution obtenus in vitro, et les cinétiques de perméation obtenues ex vivo et présentées dans les tableaux III et IV.

Enfin, on constate à nouveau que les dispositifs transdermiques matriciels selon l'invention sont remarquablement bien tolérés, selon les tests d'irritation cutanée primaire, dont les résultats figurant dans le Tableau VI ci après, et qui ont été conduits selon les termes du Journal Officiel de la République Française 1982.

**Tableau VI**

| | indice d'irritation cutanée primaire | classement |
|---|---|---|
| Ex 1 | 1,7 | légèrement irritant |
| Ex 2 | 2,0 | légèrement irritant |
| Contre Exemple 1 | 1,6 | légèrement irritant |
| Contre Exemple 3 | 3,0 | irritant |
| Comp C2 partie diffusante | 1,71 | légèrement irritant |
| Comp C2 partie adhésive | 2,42 | irritant |

On constate avantageusement que les dispositifs selon l'invention sont au moins aussi bien tolérés que le produit ANDRODERM®. Pour ce dernier produit, on constate que la périphérie adhésive ne contenant pas de principe actif est moins bien tolérée que la surface diffusante centrale. On constate également que la présence de l'adjuvant de formulation polyvinylpyrrolidone permet une meilleure tolérance cutanée de la composition telle que décrite à l'exemple 1.

## Revendications

1. Dispositif transdermique auto-adhésif, pour l'administration de testostérone et/ou d'au moins un de ses dérivés choisis parmi ses esters ou ses dérivés ayant un substituant au moins en position 6α ou 7α, **caractérisé par le fait qu'**il comporte, successivement, au moins, une couche support, une couche matrice auto-adhésive et une couche protectrice détachable, ladite couche matrice comprenant, par rapport au poids total de cette dernière:
a) 40 à 80 % en poids d'au moins un polymère auto-adhésif choisi dans le groupe constitué par les polymères de type acrylique présentant un indice d'acide compris entre 10 et 70 ayant une fonctionnalité acide et une température de transition vitreuse comprise entre - 70°C et - 20°C;
b) 5 à 25 % en poids d'au moins un adjuvant de formulation pour la couche matrice choisi parmi les polymères de masse moléculaire moyenne en nombre comprise entre 2500 et 3 000 000 ;
c) 5 à 20 % en poids d'au moins un solvant de la testostérone ou de ses dérivés choisi dans le groupe constitué par le N-diéthyl-m-toluamide, le 2-octyldodécanol, le crotamiton, le dipélargonate de propylène glycol et les mélanges de ces derniers dans lesquels la solubilité de la testostérone ou de ses dérivés est comprise entre 5 et 20% en poids;
d) 2 à 10 % en poids de testostérone et/ou d'au moins un de ses dérivés tels que définis plus haut, en solution sursaturée.

2. Dispositif selon la revendication 1, **caractérisé par le fait que** ledit polymère auto-adhésif est un polymère du monomère acide (méth)acrylique et d'au moins un monomère choisi dans le groupe constitué par les monomères (méth)acrylate d'alkyle en C1-C6, 2-(alkyl en C1-C6)hexyl(méth)acrylate, acétate de vinyle, (méth)acrylate de glycidyle, et 2-hydroxy(alkyl en C1-C6)(méth)acrylate.

3. Dispositif selon la revendication 2 **caractérisé par le fait que** ledit polymère auto-adhésif est un polymère du monomère acide (méth)acrylique et d'au moins un monomère choisi dans le groupe constitué par les monomères (méth)acrylate de méthyle, (méth)acrytate de butyle, 2-éthylhexyl(méth)acrylate, acétate de vinyle, (méth)acrylate de glycidyle, et 2-hydroxyéthyl(méth)acrylate.

4. Dispositif selon l'une quelconque des revendications précédentes, **caractérisé par le fait que** la couche matrice est réticulée.

5. Dispositif selon l'une quelconque des revendications précédentes, **caractérisé par le fait que** ledit polymère auto-adhésif est réticulé.

6. Dispositif selon l'une quelconque des revendications 1 à 3, **caractérisé par le fait que** la couche matrice n'est pas réticulée.

7. Dispositif selon l'une quelconque des revendications 1 à 3 et 6, **caractérisé par le fait que** ledit polymère auto-adhésif n'est pas réticulé.

8. Dispositif selon l'une quelconque des revendications précédentes, **caractérisé par le fait que** ledit adjuvant de formulation est choisi dans le groupe constitué par les polymères du monomère 1-vinyl-2-pyrrolydone, les polysaccharides, les dérivés cellulosiques et les mélanges de ces derniers.

9. Dispositif selon la revendication 8, **caractérisé par le fait que** ledit adjuvant de formulation est choisi dans le groupe constitué par la polyvinylpyrrolidone, les gommes xanthanes, la carboxyméthylcellulose sodique et les mélange de ces derniers.

10. Dispositif selon l'une quelconque des revendications précédentes, **caractérisé par le fait que** ledit polymère auto-adhésif est un polymère des monomères acide acrylique, 2-éthylhexylacrylate, acétate de vinyle et acrylate de butyle, ce polymère étant réticulé l'adjuvant de formulation est la polyvinylpyrrolidone de masse moléculaire moyenne en nombre comprise entre 44000 et 54000, et ledit solvant est le le N, N-diéthyl-m-toluamide.

11. Dispositif selon l'une quelconque des revendications précédentes, **caractérisé par le fait que** ladite couche matrice comprend, par rapport à son poids total :
a) 63 à 73 % en poids d'au moins un polymère auto-adhésif des monomères acide acrylique, 2-éthylhexylacrylate, acétate de vinyle et acrylate de butyle, ce polymère étant réticulé;
b) 5 à 25 % en poids de polyvinylpyrrolidone de masse moléculaire moyenne en nombre comprise entre 44000 et 54000;
c) 9 à 15 % en poids de N, N-diéthyl-m-toluamide;
d) 4 à 6 % en poids de testostérone.

12. Dispositif selon l'une quelconque des revendications précédentes, **caractérisé par le fait que** ladite couche matrice comprend en outre au moins un agent antioxydant choisi dans le groupe constitué par le butyl hydroxy Toluène (BHT), le butyl hydroxy anisole (BHA), le palmitate d'ascorbyle, l'alpha tocophérol et ses esters, l'acide citrique, le gallate de propyle et les mélanges de ces derniers.

13. Dispositif selon la revendication 12, **caractérisé par le fait que** ledit agent antioxydant est présent dans ladite couche matrice, par rapport au poids total de cette dernière, selon une proportion comprise entre 0,1 et 2 % en poids.

14. Procédé de préparation d'un dispositif transdermique selon l'une quelconque des revendications précédentes, **caractérisé en ce qu'**il comprend les étapes consistant :
a) à réaliser, dans un mélangeur, sous agitation, une solution de testostérone ou d'un de ses dérivés dans un premier solvant de fabrication, le tetrahydrofurane;
b) à incorporer, dans la solution ainsi obtenue, un deuxième solvant de fabrication choisi parmi l'éthanol, l'isopropanol ou un mélange de ces derniers ainsi que le solvant de la testostérone ou de son dérivé tel que défini à la revendication 1 destiné à être présent dans le dispositif;
c) à incorporer progressivement, sous agitation, dans un mélangeur distinct, homogénéiseur, l'adjuvant de formulation pour la couche matrice au polymère auto-adhésif; puis
d) à incorporer le mélange obtenu à la fin de l'étape b) dans celui obtenu à l'étape c) de manière à obtenir un mélange final homogène; et
e) à enduire le mélange homogène ainsi obtenu, à une température comprise entre 50°C et 110°C sur un support final de type polyester ou un support intermédiaire provisoire antiadhérent, à raison de 50 à 150 g/m², grammage mesuré sur le film sec;
f) à sécher l'enduction ainsi obtenue par séchage progressif à une température comprise entre 50°C et 110°C, afin d'évaporer les solvants de fabrication et, le cas échéant, permettre la réticulation du polymère adhésif.

15. Procédé de préparation d'un dispositif transdermique selon la revendication 14, **caractérisé en ce que** le solvant de la testostérone ou de son dérivé est la N-N-diéthyl-m-toluamide (DEET).

16. Procédé de préparation d'un dispositif transdermique selon l'une quelconque des revendications 14 ou 15, **caractérisé en ce que** l'adjuvant de formulation pour la couche matrice est la polyvinylpyrrolidone (PVP).

17. Dispositif selon l'une quelconque des revendications 1 à 13, pour son utilisation en tant que produit thérapeutiquement actif dans un médicament.

18. Utilisation du dispositif selon l'une quelconque des revendications 1 à 13, pour la préparation d'un médicament destiné à une thérapie de remplacement de testostérone.

19. Utilisation selon la revendication 18, **caractérisée en ce que** le médicament est destiné à une thérapie journalière, hebdomadaire ou bi hebdomadaire de remplacement de testostérone.

20. Utilisation selon la revendication 18 ou 19, **caractérisée par le fait que** le médicament est destiné au traitement et/ou à la prévention de l'hypogonadisme.

21. Utilisation selon la revendication 18 ou 19, **caractérisée par le fait que** le médicament est destiné au traitement et/ou à la prévention des troubles de l'andropause.

22. Utilisation selon la revendication 18 ou 19, **caractérisée par le fait que** le médicament est destiné au traitement et/ou à la prévention de l'impuissance sexuelle.

23. Utilisation selon la revendication 18 ou 19, **caractérisée par le fait que** le médicament est destiné au traitement et/ou à la prévention des troubles de la fertilité chez l'homme.

24. Utilisation selon la revendication 18 ou 19, **caractérisée par le fait que** le médicament est destiné au traitement et/ou à la prévention de l'oligospermie.

25. Utilisation selon la revendication 18 ou 19, **caractérisée par le fait que** le médicament est destiné au traitement et/ou à la prévention de l'éjaculation précoce.

## Patentansprüche

1. Selbstklebende transdermale Vorrichtung für die Verabreichung von Testosteron und/oder mindestens eines seiner Derivate, die aus seinen Estern oder seinen Derivaten mit einem Substituenten mindestens in der Position 6α oder 7α ausgewählt sind, **dadurch gekennzeichnet, dass** sie aufeinanderfolgend mindestens eine Trägerschicht, eine selbstklebende Matrixschicht und eine entfernbare Schutzschicht umfasst, wobei die Matrixschicht, bezogen auf das Gesamtgewicht der Letztgenannten, umfasst:
a) 40 bis 80 Gew.-% mindestens eines selbstklebenden Polymers, das aus der Gruppe ausgewählt ist, die aus Polymeren vom Acryl-Typ besteht, die eine Säurezahl zwischen 10 und 70 einschließlich aufweisen und eine saure Funktionalität und eine Glasübergangstemperatur zwischen - 70 °C und -20 °C einschließlich aufweisen;
b) 5 bis 25 Gew.-% mindestens eines Formulierungshilfsmittels für die Matrixschicht, das aus Polymeren mit einem Zahlenmittel des Molekulargewichts zwischen 2 500 und 3 000 000 einschließlich ausgewählt ist;
c) 5 bis 20 Gew.-% mindestens eines Lösungsmittels für das Testosteron oder seine Derivate, das aus der Gruppe ausgewählt ist, die aus N-Diethyl-m-toluamid, 2-Octyldodecanol, Crotamiton, dem Dipelargonat von Propylenglycol und den Mischungen der Letztgenannten besteht, in denen die Löslichkeit des Testosterons oder seiner Derivate zwischen 5 und 20 Gew.-% einschließlich liegt;
d) 2 bis 10 Gew.-% Testosteron und/oder mindestens eines seiner Derivate, wie oben definiert, in übersättigter Lösung.

2. Vorrichtung nach Anspruch 1, **dadurch gekennzeichnet, dass** das selbstklebende Polymer ein Polymer des Monomers (Meth)acrylsäure und mindestens eines Monomers ist, das aus der Gruppe ausgewählt ist, die aus den Monomeren (C₁ - C₆)-Alkyl(meth)acrylat, 2-((C₁ - C₆)-Alkyl)hexyl(meth)acrylat, Vinylacetat, Glycidyl(meth)acrylat und 2-Hydroxy((C₁ - C₆)-alkyl)(meth)acrylat besteht.

3. Vorrichtung nach Anspruch 2, **dadurch gekennzeichnet, dass** das selbstklebende Polymer ein Polymer des Monomers (Meth)acrylsäure und mindestens eines Monomers ist, das aus der Gruppe ausgewählt ist, die aus den Monomeren Methyl(meth)acrylat, Butyl(meth)acrylat, 2-Ethylhexyl(meth)acrylat, Vinylacetat, Glycidyl(meth)acrylat und 2-Hydroxyethyl(meth)-acrylat besteht.

4. Vorrichtung nach irgendeinem der vorangehenden Ansprüche, **dadurch gekennzeichnet, dass** die Matrixschicht vernetzt ist.

5. Vorrichtung nach irgendeinem der vorangehenden Ansprüche, **dadurch gekennzeichnet, dass** das selbstklebende Polymer vernetzt ist.

6. Vorrichtung nach irgendeinem der Ansprüche 1 bis 3, **dadurch gekennzeichnet, dass** die Matrixschicht nicht vernetzt ist.

7. Vorrichtung nach irgendeinem der Ansprüche 1 bis 3 und 6, **dadurch gekennzeichnet, dass** das selbstklebende Polymer nicht vernetzt ist.

8. Vorrichtung nach irgendeinem der vorangehenden Ansprüche, **dadurch gekennzeichnet, dass** das Formulierungshilfsmittel aus der Gruppe ausgewählt ist, die aus Polymeren des Monomers 1-Vinyl-2-pyrrolidon, Polysacchariden, Cellulose-Derivaten und den Mischungen dieser Letztgenannten besteht.

9. Vorrichtung nach Anspruch 8, **dadurch gekennzeichnet, dass** das Formulierungshilfsmittel aus der Gruppe ausgewählt ist, die aus Polyvinylpyrrolidon, Xanthangummis, Natriumcarboxymethylcellulose und den Mischungen der Letztgenannten besteht.

10. Vorrichtung nach irgendeinem der vorangehenden Ansprüche, **dadurch gekennzeichnet, dass** das selbstklebende Polymer ein Polymer der Monomere Acrylsäure, 2-Ethylhexylacrylat, Vinylacetat und Butylacrylat ist, wobei dieses Polymer vernetzt ist, das Formulierungshilfsmittel Polyvinylpyrrolidon mit einem Zahlenmittel des Molekulargewichts zwischen 44 000 und 54 000 einschließlich ist und das Lösungsmittel N,N-Diethyl-m-toluamid ist.

11. Vorrichtung nach irgendeinem der vorangehenden Ansprüche, **dadurch gekennzeichnet, dass** die Matrixschicht, bezogen auf ihr Gesamtgewicht, umfasst:
a) 63 bis 73 Gew.-% mindestens eines selbstklebenden Polymers der Monomere Acrylsäure, 2-Ethylhexylacrylat, Vinylacetat und Butylacrylat, wobei dieses Polymer vernetzt ist;
b) 5 bis 25 Gew.-% Vinylpyrrolidon mit einem Zahlenmittel des Molekulargewichts zwischen 44 000 und 54 000 einschließlich;
c) 9 bis 15 Gew.-% N,N-Diethyl-m-toluamid;
d) 4 bis 6 Gew.-% Testosteron.

12. Vorrichtung nach irgendeinem der vorangehenden Ansprüche, **dadurch gekennzeichnet, dass** die Matrixschicht darüber hinaus mindestens ein Antioxidans umfasst, das aus der Gruppe ausgewählt ist, die aus Butylhydroxytoluol (BHT), Butylhydroxyanisol (BHA), Ascorbylpalmitat, alpha-Tocopherol und dessen Estern, Citronensäure, Propylgallat und Mischungen der Letztgenannten besteht.

13. Vorrichtung nach Anspruch 12, **dadurch gekennzeichnet, dass** das Antioxidans in der Matrixschicht, bezogen auf das Gesamtgewicht der Letztgenannten, in einem Verhältnis zwischen 0,1 und 2 Gew.-% einschließlich vorliegt.

14. Verfahren zur Herstellung einer transdermalen Vorrichtung nach irgendeinem der vorangehenden Ansprüche, **dadurch gekennzeichnet, dass** es die Schritte umfasst, die bestehen aus:
a) Herstellung einer Lösung von Testosteron oder eines seiner Derivate in einem ersten Herstellungslösungsmittel, Tetrahydrofuran, unter Rühren in einem Mischer;
b) Einbringen eines zweiten Herstellungslösungsmittels, das aus Ethanol, Isopropanol oder einer Mischung dieser Letztgenannten ausgewählt ist, sowie des Lösungsmittels für das Testosteron oder sein Derivat, wie in Anspruch 1 definiert, das dazu bestimmt ist, in der Vorrichtung anwesend zu sein, in die so erhaltene Lösung;
c) fortschreitendes Einbringen des Formulierungshilfsmittels für die Matrixschicht mit dem selbstklebendem Polymer unter Rühren in einen getrennten Homogenisierungsmischer; dann
d) Einbringen der am Ende von Schritt b) erhaltenen Mischung in die im Schritt c) erhaltene auf solche Weise, dass man eine homogene Endmischung erhält; und
e) Auftragen der so erhaltenen homogenen Mischung bei einer Temperatur zwischen 50 °C und 110 °C einschließlich auf einen Endträger vom Polyester-Typ oder einen provisorischen nicht-haftenden Zwischenträger mit einem Verhältnis von 50 bis 150 g/m² Flächengewicht, gemessen beim trockenen Film;
f) Trocknen der so erhaltenen Schicht durch fortschreitendes Trocknen bei einer Temperatur zwischen 50 °C und 110 °C einschließlich, um die Herstellungslösungsmittel zu verdampfen und gegebenenfalls die Vernetzung des klebenden Polymers zu ermöglichen.

15. Verfahren zur Herstellung einer transdermalen Vorrichtung nach Anspruch 14, **dadurch gekennzeichnet, dass** das Lösungsmittel für das Testosteron oder sein Derivat N,N-Diethyl-m-toluamid (DEET) ist.

16. Verfahren zur Herstellung einer transdermalen Vorrichtung nach einem der Ansprüche 14 oder 15, **dadurch gekennzeichnet, dass** das Formulierungshilfsmittel für die Matrixschicht Polyvinylpyrrolidon (PVP) ist.

17. Vorrichtung nach irgendeinem der Ansprüche 1 bis 13 zur Verwendung als therapeutisch aktives Produkt in einem Medikament.

18. Verwendung der Vorrichtung nach irgendeinem der Ansprüche 1 bis 13 für die Herstellung eines Medikaments, das für eine Therapie des Testosteron-Ersatzes bestimmt ist.

19. Verwendung nach Anspruch 18, **dadurch gekennzeichnet, dass** das Medikament für eine tägliche, wöchentliche oder zweiwöchentliche Therapie des Testosteron-Ersatzes bestimmt ist.

20. Verwendung nach Anspruch 18 oder 19, **dadurch gekennzeichnet, dass** das Medikament zur Behandlung und/oder Verhütung von Hypogonadismus bestimmt ist.

21. Verwendung nach Anspruch 18 oder 19, **dadurch gekennzeichnet, dass** das Medikament zur Behandlung und/oder Verhütung von Beschwerden der Andropause bestimmt ist.

22. Verwendung nach Anspruch 18 oder 19, **dadurch gekennzeichnet, dass** das Medikament zur Behandlung und/oder Verhütung von sexueller Impotenz bestimmt ist.

23. Verwendung nach Anspruch 18 oder 19, **dadurch gekennzeichnet, dass** das Medikament zur Behandlung und/oder Verhütung von Störungen der Fruchtbarkeit beim Mann bestimmt ist.

24. Verwendung nach Anspruch 18 oder 19, **dadurch gekennzeichnet, dass** das Medikament zur Behandlung und/oder Verhütung von Oligospermie bestimmt ist.

25. Verwendung nach Anspruch 18 oder 19, **dadurch gekennzeichnet, dass** das Medikament zur Behandlung und/oder Verhütung von Ejaculatio praecox bestimmt ist.

## Claims

1. Self-adhesive transdermal device for administering testosterone and/or at least one derivative thereof chosen from the esters or derivatives thereof housing a substituent at least in the 6α-or 7α-position, **characterized in that** it comprises, successively, at least, one support layer, one self-adhesive matrix layer and one detachable protective layer, said matrix layer comprising, relative to the total weight of said detachable protective layer:
a) 40% to 80% by weight of at least one self-adhesive polymer chosen from the group consisting of polymers of acrylic type an acid number of between 10 and 70 with an acid functionality and a glass transition temperature of between -70°C and -20°C;
b) 5% to 25% by weight of at least one formulation adjuvant for the matrix layer chosen from polymers with a number-average molecular mass of between 2 500 and 3 000 000;
c) 5% to 20% by weight of at least one solvent for testosterone or derivatives thereof chosen from the group consisting of N,N-diethyl-m-toluamide, 2-octyldodecanol, crotamiton and propylene glycol dipelargonate, and mixtures thereof, in which the solubility of the testosterone or derivatives thereof is between 5% and 20% by weight;
d) 2% to 10% by weight of testosterone and/or of at least one derivative thereof as defined above, as a supersaturated solution.

2. Device according to Claim 1, **characterized in that** said self-adhesive polymer is a polymer of (meth)acrylic acid monomer and of at least one monomer chosen from the group consisting of C1-C6 alkyl (meth)acrylate, 2-(C1-C6 alkyl)hexyl (meth)acrylate, vinyl acetate, glycidyl (meth)acrylate and 2-hydroxy(C1-C6 alkyl) (meth)acrylate monomers.

3. Device according to Claim 2, **characterized in that** said self-adhesive polymer is a polymer of (meth)acrylic acid monomer and of at least one monomer chosen from the group consisting of methyl (meth)acrylate, butyl (meth)acrylate, 2-ethylhexyl (meth)acrylate, vinyl acetate, glycidyl (meth)acrylate and 2-hydroxyethyl (meth)acrylate monomers.

4. Device according to any one of the preceding claims, **characterized in that** the matrix layer is crosslinked.

5. Device according to any one of the preceding claims, **characterized in that** said self-adhesive polymer is crosslinked.

6. Device according to any one of Claims 1 to 3, **characterized in that** the matrix layer is not crosslinked.

7. Device according,to any one of Claims 1 to 3 and 6, **characterized in that** said self-adhesive polymer is not crosslinked.

8. Device according to any one of the preceding claims, **characterized in that** said formulation adjuvant is chosen from the group consisting of polymers of 1-vinyl-2-pyrrolidone monomer, polysaccharides and cellulose derivatives, and mixtures thereof.

9. Device according to Claim 8, **characterized in that** said formulation adjuvant is chosen from the group consisting of polyvinylpyrrolidone, xanthan gums and sodium carboxymethylcellulose, and mixtures thereof.

10. Device according to any one of the preceding claims, **characterized in that** said self-adhesive polymer is a polymer of acrylic acid, 2-ethylhexyl acrylate, vinyl acetate and butyl acrylate monomers, this polymer being crosslinked, the formulation adjuvant is polyvinylpyrrolidone with a number-average molecular mass of between 44 000 and 54 000, and the solvent is N,N-diethyl-m-toluamide.

11. Device according to any one of the preceding claims, **characterized in that** said matrix layer comprises, relative to its total weight:
a) 63% to 73% by weight of at least one self-adhesive polymer of acrylic acid, 2-ethylhexyl acrylate, vinyl acetate and butyl acrylate monomers, this polymer being crosslinked;
b) 5% to 25% by weight of polyvinylpyrrolidone with a number-average molecular mass of between 44 000 and 54 000;
c) 9% to 15% by weight of N,N-diethyl-m-toluamide;
d) 4% to 6% by weight of testosterone.

12. Device according to any one of the preceding claims, **characterized in that** said matrix layer also comprises at least one antioxidant chosen from the group consisting of butylhydroxytoluene (BHT), butylhydroxyanisole (BHA), ascorbyl palmitate, α-tocopherol and its esters, citric acid and propyl gallate, and mixtures thereof.

13. Device according to Claim 12, **characterized in that** said antioxidant is present in said matrix layer, relative to the total weight of said matrix layer, in a proportion of between 0.1% and 2% by weight.

14. Process for preparing a transdermal device according to any one of the preceding claims, **characterized in that** it comprises the steps consisting:
a) in preparing, in a mixer, with stirring, a solution of testosterone or a derivative thereof in a first manufacturing solvent which is tetrahydrofuran;
b) in incorporating, into the solution thus obtained, a second manufacturing solvent chosen from ethanol and isopropanol, or a mixture thereof and also the solvent for testosterone or its derivative as defined in Claim 1, which is intended to be present in the device;
c) in gradually incorporating, with stirring, in a separate mixer of homogenizer, the formulation adjuvant for the matrix layer into the self-adhesive polymer; and then
d) in incorporating the mixture obtained at the end of step b) into that obtained in step c) so as to obtain a final homogeneous mixture; and
e) in coating the homogeneous mixture thus obtained, at a temperature of between 50°C and 110°C, onto a final support of polyester type or an antiadhesive temporary intermediate support, at a rate of from 50 to 150 g/m², this weight per unit area being measured on the dry film;
f) in drying the coating thus obtained, by gradual drying at a temperature of between 50°C and 110°C, so as to evaporate the manufacturing solvents and, where appropriate, to allow the crosslinking of the adhesive polymer.

15. Process for preparing a transdermal device according to Claim 14, **characterized in that** the solvent for testosterone or its derivative is N,N-diethyl-m-toluamide (DEET).

16. Process for preparing a transdermal device according to either of Claims 14 and 15, **characterized in that** the formulation adjuvant for the matrix layer is polyvinylpyrrolidone (PVP).

17. Device according to any one of Claims 1 to 13, for its use as a therapeutically active product in a medicinal product.

18. Use of the device according to any one of Claims 1 to 13, for preparing a medicinal product intended for a testosterone replacement therapy.

19. Use according to Claim 18, **characterized in that** the medicinal product is intended for a daily, weekly or twice-weekly testosterone replacement therapy.

20. Use according to Claim 18 or 19, **characterized in that** the medicinal product is intended for treating and/or preventing hypogonadism.

21. Use according to Claim 18 or 19, **characterized in that** the medicinal product is intended for treating and/or preventing andropause disorders.

22. Use according to Claim 18 or 19, **characterized in that** the medicinal product is intended for treating and/or preventing sexual impotence.

23. Use according to Claim 18 or 19, **characterized in that** the medicinal product is intended for treating and/or preventing fertility disorders in men.

24. Use according to claim 18 or 19, **characterized in that** the medicinal product is intended for treating and/or preventing oligospermy.

25. Use according to Claim 18 or 19, **characterized in that** the medicinal product is intended for treating and/or preventing premature ejaculation.
